# EUROPÄISCHE PATENTSCHRIFT

(11) **EP 3 159 017 B1**
(45) Veröffentlichungstag und Bekanntmachung des Hinweises auf die Patenterteilung: **02.05.2018**
(21) Anmeldenummer: 15002972.6
(22) Anmeldetag: 19.10.2015
(51) Int. Cl.: A61L 27/18, A61L 27/38, A61L 27/56, A61L 27/58, A61L 27/60

(54) **RESORBIERBARE MATRIX ZUR WUNDABDECKUNG**
RESORBABLE MATRIX FOR WOUND COVERINGS
MATRICE RESORBABLE DESTINEE AU RECOUVREMENT DE PLAIE

(43) Veröffentlichungstag der Anmeldung: 26.04.2017
(73) Patentinhaber: Bioenergy Capital AG, 50668 Köln (DE)
(72) Erfinder: Schröter, Guido, 13505 Berlin (DE); Roloff, Frank, 30926 Seelze (DE)
(74) Vertreter: Diehl & Partner GbR

(56) Entgegenhaltungen:
- WO-A1-2013/093921
- HOLY C E ET AL: "Optimizing the sterilization of PLGA scaffolds for use in tissue engineering", BIOMATERIALS, ELSEVIER SCIENCE PUBLISHERS BV., BARKING, GB, Bd. 22, Nr. 1, 1. Januar 2000 (2000-01-01) , Seiten 25-31, XP004218037, ISSN: 0142-9612, DOI: 10.1016/S0142-9612(00)00136-8
- TIM DESMET ET AL: "Nonthermal Plasma Technology as a Versatile Strategy for Polymeric Biomaterials Surface Modification: A Review", BIOMACROMOLECULES, Bd. 10, Nr. 9, 14. September 2009 (2009-09-14), Seiten 2351-2378, XP055260902, US ISSN: 1525-7797, DOI: 10.1021/bm900186s

## Beschreibung

Die vorliegende Anmeldung bezieht sich auf poröse Matrices für Zwecke der Wundabdeckung.

Zellimplantate auf der Basis poröser Matrices aus bioverträglichen Polymeren zur Verwendung in der Geweberekonstruktion sind aus US 2009/0291116 A1 bekannt. Bei solchen Matrices wird ein Templat für die Ansiedlung von Zellen aus verschiedenen, nämlich hydrophileren und weniger hydrophilen Polymerblöcken komponiert. Die Porosität wird dadurch erreicht, dass man bei der Herstellung das Polymer mit einer Salzmischung (z. B. NaCl/NH₄HCO₃) im Überschuss versetzt und letztere danach im warmen Wasserbad auslaugt.

Die bekannten Template sind noch nicht voll befriedigend, insbesondere hinsichtlich der mechanischen Eigenschaften: Vielfach sind die erhaltenen Matrices zu fragil für eine einfache Handhabung oder gar den chirurgischen Einsatz, und außerdem sind die Abmessungen schwer zu steuern, weil sie sich während des Herstellungsverfahrens stark ändern.

Der Artikel *"*Optimizing the sterilization of PLGA scaffolds for use in tissue engineering" von Holy et al. in Biomaterials 22 (2001), Seiten 22-31, offenbart ein Verfahren der Sterilisation von in einem Sterilisierbeutel verpackten, säulenförmigen Osteofoam®-Werkstücken in einem Argon-Plasma bei 100 W für 4 -Minuten. Die Werkstücke selbst werden durch Herauslösen von Glukosekristallen mittels entionisiertem destilliertem Wasser aus einer Matrix aus durch Abscheiden aus DMSO erhaltenem PLGA 75/25-Copolymer hergestellt.

Die Erfindung setzt sich daher zum Ziel, eine Verbesserung der mechanischen Eigenschaften der bekannten Template zur Wundabdeckung zu erreichen.

Dazu schlägt die Erfindung Verfahren gemäß den Ansprüchen 1 oder 4 zur Herstellung von porösen bioresorbierbaren Matrices zur Wundabdeckung vor, wobei ein Gemisch aus resorbierbarem Polymer, einem wasserlöslichen Porenbildner und einem Lösungsmittel für das Polymer hergestellt wird, gefolgt von Abdampfen des Lösungsmittels und Wässern zur Porenbildung. Vor dem Abdampfungsschritt wird die Masse in eine zweiteilige Form mit abnehmbaren Rand, oder auf einen wasserdurchlässigen Träger gebracht. Zum Wässern wird der ringförmige Rand von der Bodenplatte abgehoben, bzw. der wasserdurchlässige Träger z. B auf einem Gestell in einer Wanne gelagert. Auf beide Weisen erfolgt der Wasserzutritt und vor allem der Abtransport der beim Laugungsprozess entstehenden Salzlake erheblich beschleunigt, und die Matrix behält dabei ihre Struktur und ihre mechanischen Eigenschaften weitgehend.

In Ausführungsformen wird die gewässerte und dann luftgetrocknete Matrix ohne irgendeine Beschichtung in einem Sterilisationsbeutel mit zumindest einer mikroporösen Seite verpackt und darin mittels eines Plasmaverfahrens sterilisiert, ggf. unter Zutritt von H₂O₂. Die Erfinder haben gefunden, dass die so behandelte Matrix überraschenderweise nicht nur steril, sondern auch ausreichend hydrophil ist, um im therapeutischen Einsatz von infiltrierenden Zellen nachhaltig besiedelt zu werden.

In Ausführungsformen ist das bioresorbierbare Polymer bzw. sind die bioresorbierbaren Polymere solche, die nominell innerhalb von ein bis drei Monaten resorbiert werden. Innerhalb dieser Zeit bildet sich ausreichend neues Gewebe aus infiltrierenden, in den untereinander verbundenen Poren der nach und nach resorbiert werdenden Matrix proliferierenden Zellen.

Unter einem weiteren Aspekt schlägt die Erfindung als Templat eine poröse Matrix aus einem, ggf. einem Gemisch von resorbierbaren Polymeren vor, insbesondere wie nach dem oben beschriebenen Verfahren erhältlich; wobei die poröse Matrix in einem Beutel mit zumindest einer mikroporösen Seite verpackt ist, deren Porengröße 50 µm oder weniger, insbesondere 25 µm oder weniger beträgt.

In der Hauptanwendung werden Matrices gemäß Anspruch 10 zur Wundabdeckung bereitgestellt. Die verwendeten Polymere sind zweckmäßig solche auf Basis von α-Hydroxycarbonsäuren wie Milchsäure und/oder Glykolsäure, z. B. PLA oder PLGA. Die Hersteller solcher zum Einsatz im menschlichen Körper zugelassener Polymere geben die hier relevanten nominellen Abbauzeiten an. Die hier verwendeten Polymere sind z. B. von der Fa. Evonik erhältlich und tragen die Bezeichnungen RG502 und RG505 (innerhalb von 3 Monaten abbaubare PLGA-Polymere). Auch Dioxanon, Trimethylencarbonat und ε-Caprolacton können als Polymer oder als Copolymer eingesetzt werden.

Die erfindungsgemäßen Matrices werden mechanisch hinreichend stabil hergestellt, dass sie z. B. den Belastungen durch chirurgische Nähvorgänge standhalten. An ihrer Peripherie können die Matrices daher mit Körpergewebe verbunden, beispielsweise vernäht werden. Ihre Porosität stellt sicher, dass die Matrices von Hautzellen infiltriert und vaskularisiert werden können.

Gemäß einer Ausführungsform weist die Matrix eine porenärmere Seite auf, die für die eigentliche Abdeckung sorgt, und eine porenreichere, die für die Infiltration günstig ist. Die glattere porenarme Seite kann dem Körperäußeren zugewandt angeordnet werden, um bei Kontakt z. B. mit Verbandsmaterial weniger Reibung zu verursachen, welche sich belastend auf die Wundränder auswirken könnte.

Die Matrix kann, muss aber nicht vor dem Einsatz mit Zellen, beispielsweise Keratinozyten infiltriert werden; idealerweise mit körpereigenen. Solche Zellen heften sich an die Innenwände der Poren der Matrix an (mit Anheftungsraten von über 80 % oder sogar über 95 %) und können mit der Matrix implantiert werden. Dabei wird ausgenutzt, dass bei körpereigenen Zellen keine Abstoßungsreaktion erfolgt, sondern nur ein vergleichsweise milder, für den therapeutischen Prozess günstiger Fremdkörper-Reiz ausgeübt wird. Binnen weniger Wochen wird die Matrix vaskularisiert und sind die implantierten und die infiltrierenden Zellen nicht mehr nur auf diffusive Versorgung angewiesen und proliferieren. Gleichzeitig wird die Matrix resorbiert, so dass danach die behandelte Stelle mit eigenem Hautgewebe bedeckt ist, ohne dass eingesetztes Material entfernt werden müsste (ausgenommen selbstverständlich bloßes Verbandsmaterial).

Zur Herstellung wird in einer Ausführungsform eine Polymer-Granulat mit einem Kochsalz-Granulat gemischt, mit einer Lösung des Polymers bzw. eines der Polymere in Chloroform vermengt und dann auf die bereitgestellte wasserdurchlässige Trägerschicht oder in eine Form mit abnehmbarem Rand gegeben. Aus diesem Formling dampft das Lösungsmittel bei leicht erhöhter Temperatur (30-45°C) oder entsprechend leicht reduziertem Druck ab; währenddessen kann er gewünschtenfalls durch Beaufschlagung mit Druck kompaktiert werden. Anschließend wird der Kompaktkörper gewässert, um durch Entfernen des Salzes die gewünschte Porosität bereitzustellen. Dazu wird je nach Variante der Rand der Form, an dem die Matrix anhaftet, von deren Boden abgehoben, und es wird (in einer Wanne) Wasser zugegeben, bzw. der wasserdurchlässige Träger wird mitsamt der auflagernden Matrix in ein Wasserbad gestellt. Der Rand der Form kann beispielsweise durch einen Metallring bereitgestellt sein. Der Rand kann die Form eines Zylindermantels oder Kegelstumpfmantels aufweisen. In beiden Varianten hat das Wasser des Bads beidseitigen Zutritt zur Matrix, und das Auslaugen erfolgt schneller als dies bei nur einseitigem Zutritt der Fall wäre. Zweckmäßig wird das Wasser von oberhalb einer Mittenebene der flächigen Matrix zu-, und die Salzlake ihrer größeren Dichte wegen nach unterhalb der Mittenebene abgeführt. Dadurch wird eine unerwünschte Vermischung beider weitgehend vermieden.

Der in der einen Variante verwendete Träger besteht z. B. aus einer Platte aus hydrophilisiertem, gegen Chloroform beständigem und mit Durchgangsporen versehenem Kunststoff (z. B. Teflon). Die Poren können einen Durchmesser von bis zu, oder weniger als 0,5 mm aufweisen. Ein solcher Träger ist für das Polymer-/Salzgemisch undurchlässig, lässt aber den Durchtritt von Wasser oder Salzlake zu, wenn er, z. B. auf einem Drahtgestell, in eine mit Wasser gefüllte Wanne gestellt wird. In der anderen Variante kann es zweckmäßig sein, als Boden eine Teflon-beschichtete Glasplatte oder dergleichen zu verwenden, um sicherzustellen, dass die Polymer-/Salzmasse nicht daran, sondern nur am Ring haftet und sich leicht vom Boden der Form löst.

Das Wasser zum Auslaugen wird in zunächst kürzeren, dann längeren Zeitabständen gewechselt; in einer Variante wird durch kontinuierliches Zu- und Abführen ein ständiger Austausch vollzogen. In Ausführungsformen reicht dafür eine Zeit von 2-6 Stunden aus. Dabei wird die Matrix nur unwesentlich hydrolysiert und behält ihre Abmessungen und mechanischen Eigenschaften.

Die Kochsalzpartikel der porenbildenden Mischung sind etwas gröber (Median bei 200-400 µm) als die Polymerpartikel (Median zwischen 150 µm und 200 µm). Dabei sind die Verteilungsbreiten (5 %/95 %) ähnlich, nämlich etwa ± 85-95 µm für Salz bzw. Polymer insgesamt. Die Verteilungsform kann bi- oder trimodal sein. Die Zusammensetzung der Schichtmischung ist zu 80-96 % Salz, 1-5 % festes Polymer und weitere ca. 3-15 % gelöstes Polymer. Das Wässern dauert nur ca. 2-6 h und wird gefolgt von einer Trocknung bei 30-45°C. Zweckmäßige Schichtdicken der Matrix liegen insbesondere im Bereich 0,5 mm-4 mm, betragen also mehr als die 95% kleinsten Salzpartikel (nach Masse) als Durchmesser aufweisen. Dadurch wird gewährleistet, dass zwar die Poren zusammenhängen, es jedoch keine oder nur sehr wenige Durchgangslöcher in der Matrix gibt, durch die hindurch das darunterliegende Gewebe äußeren Einflüssen sonst direkt ausgesetzt wäre.

Bei einer besonderen Anwendung wird die hergestellte Matrix mit primären Zellen oder Zelllinien, oder auch Zell- oder Gewebebestandteilen infiltriert und auf die Läsion gelegt, ggf. mit dem umliegenden, intakten Hautgewebe vernäht, und dann mit Verbandmaterial abgedeckt. Nach Ablauf der zur Resorption des bzw. der Polymere erforderlichen Zeit hat sich neues Hautgewebe gebildet und die ursprüngliche Matrix durch- und überwachsen.

Bei einer anderen Anwendung unterlässt man die Vorab-Infiltration, sondern setzt die hergestellte Matrix als Platzhalter für sich aus in der Umgebung vorhandenen Haut- und Endothelzellen neu bildendes Gewebe ein. In beiden Fällen dient die poröse Polymermatrix vermittels ihrer Resorption auch dem therapeutischen Geschehen, ist also Agens dieser Behandlung.

## Patentansprüche

1. Verfahren zur Herstellung einer porösen Matrix zur Wundabdeckung aus einem bioabbaubaren Polymer oder einem Gemisch von mehreren bioabbaubaren Polymeren, wobei Partikel des bzw. der Polymere mit Partikeln eines wasserlöslichen Feststoffs und einer Lösung wenigstens eines Teils des bzw. der Polymeren in einem mit Wasser nicht mischbaren Lösungsmittel gemischt werden, die Mischung auf einem wasserdurchlässigen Träger gelagert, das Lösungsmittel abgedampft und der Feststoff durch Wässern entfernt wird, wonach die ausgelaugte Matrix getrocknet und ohne eine Beschichtung in einem Beutel mit zumindest einer mikroporösen Seite verpackt und darin durch Plasmaeinwirkung sterilisiert und hydrophilisiert wird.

2. Verfahren nach Anspruch 1, wobei der wasserdurchlässige Träger zum Wässern auf einem Gestell in einem Wasserbad angeordnet wird.

3. Verfahren nach Anspruch 1 oder 2, wobei der wasserdurchlässige Träger aus einer hydrophilen und lösemittelbeständigen Kunststoffplatte mit durchgehenden Poren besteht.

4. Verfahren zur Herstellung einer porösen Matrix zur Wundabdeckung aus einem bioabbaubaren Polymer oder einem Gemisch von mehreren bioabbaubaren Polymeren, wobei Partikel des bzw. der Polymere mit Partikeln eines wasserlöslichen Feststoffs und einer Lösung wenigstens eines Teils des bzw. der Polymeren in einem mit Wasser nicht mischbaren Lösungsmittel gemischt werden, die Mischung in eine Form mit abnehmbarem Rand eingebracht wird, das Lösungsmittel abgedampft und der Rand mit der daran anhaftenden Masse vom Boden der Form abgehoben, und der Feststoff dann durch Wässern entfernt wird, wonach die ausgelaugte Matrix getrocknet und ohne eine Beschichtung in einem Beutel mit zumindest einer mikroporösen Seite verpackt und darin durch Plasmaeinwirkung sterilisiert und hydrophilisiert wird.

5. Verfahren nach Anspruch 4, wobei der abnehmbare Rand einen Metallring umfasst.

6. Verfahren nach Anspruch 4 oder 5, wobei der abnehmbare Rand eine Zylindermantel- oder Kegelstumpfmantelform aufweist.

7. Verfahren nach einem der vorstehenden Ansprüche, wobei das Wässern die kontinuierliche Zufuhr von Wasser und Abfuhr von Salzlake umfasst.

8. Verfahren nach einem der vorstehenden Ansprüche, wobei das Plasma in einer Gasmischung erzeugt wird, die H₂O₂ enthält, und wobei die Gasmischung die mikroporöse Seite des Beutels durchdringt.

9. Verfahren nach einem der vorstehenden Ansprüche, wobei die Poren des Beutels kleiner als 0,05 mm oder kleiner als 0,025 mm sind.

10. Bioabbaubare/s Polymer/e zur Anwendung in einem Verfahren der Wundabdeckung und ggf. Haut-Rekonstruktion in Form einer porösen Matrix, bestehend aus dem oder den mehreren bioabbaubaren Polymeren, jedoch ohne Beschichtung, erhältlich nach dem Verfahren gemäß einem der vorstehenden Ansprüche, wobei die Matrix flächig und zur Wundabdeckung geeignet ist.

11. Bioabbaubare/s Polymer/e zur Anwendung nach Anspruch 10, wobei die Oberfläche der Matrix durch eine Plasmabehandlung hydrophilisiert ist.

12. Bioabbaubare/s Polymer/e zur Anwendung nach Anspruch 10 oder 11, wobei die Matrix eine Porosität von mindestens 80 % oder mindestens 90% aufweist.

13. Bioabbaubare/s Polymer/e zur Anwendung nach einem der Ansprüche 10 bis 12, wobei die nominelle Resorptionszeit des bzw. wenigstens eines der Polymeren weniger als 3 Monate beträgt.

14. Bioabbaubare/s Polymer/e zur Anwendung nach einem der Ansprüche 10 bis 13, wobei das bzw. die Polymere aus Poly(α-hydroxy)carbonsäuren wie PLA und PLGA ausgewählt sind.

15. Bioabbaubare/s Polymer/e zur Anwendung nach einem der Ansprüche 10 bis 14, wobei die Matrix bis zur Anwendung in einem Beutel mit zumindest einer mikroporösen Seite verpackt ist, deren Porengröße 0,05 mm oder weniger beträgt.

16. Bioabbaubare/s Polymer/e zur Anwendung nach einem der Ansprüche 10 bis 15, infiltriert mit primären Zellen oder Zelllinien, Zell- oder Gewebebestandteilen, zur Anwendung in einem Verfahren der Haut-Rekonstruktion.

## Claims

1. A method for manufacturing a porous matrix for wound dressing made of a biodegradable polymer or a mixture of plural biodegradable polymers, wherein particles of the polymer or the polymers, respectively, are mixed with particles of a water-soluble solid and a solution of at least part of the polymer resp. polymers in a solvent immiscible with water, the mixture is layered on a water-pervious carrier, the solvent is evaporated, and the solid is removed by watering, whereafter the eluted matrix is dried and packaged without any coating in a bag having at least one microporous side, and is sterilised and hydrophilised therein by plasma action.

2. The method of claim 1, wherein the water-pervious carrier, for the watering, is arranged on a rack in a water bath.

3. The method of claim 1 or 2, wherein the water-pervious carrier consists of a hydrophilic and solvent resistant plastic plate with through-going pores.

4. A method for manufacturing a porous matrix for wound dressing made of a biodegradable polymer or a mixture of plural biodegradable polymers, wherein particles of the polymer or the polymers, respectively, are mixed with particles of a water-soluble solid and a solution of at least part of the polymer resp. polymers in a solvent immiscible with water, the mixture is brought into a mold having a detachable rim, the solvent is evaporated and the rim is lifted off the bottom of the mold together with the mass adhering thereto, and the solid is then removed by watering, whereafter the eluted matrix is dried and packaged without any coating in a bag having at least one microporous side, and is sterilised and hydrophilised therein by plasma action.

5. The method of claim 4, wherein the detachable rim includes a metal ring.

6. The method of claim 4 or 5, wherein the detachable rim has cylinder wall shape or truncated cone wall shape.

7. The method of one of the preceding claims, wherein the watering includes the continuous supply of water and discharge of saline.

8. The method of one of the preceding claims, wherein the plasma is generated in a gas mixture comprising H₂O₂, and wherein the gas mixture penetrates the microporous side of the bag.

9. The method of one of the preceding claims, wherein the pores of the bag are smaller than 0.05 mm or smaller than 0.025 mm.

10. A biodegradable polymer/polymers for use in a method of wound dressing and optionally skin reconstruction in the shape of a porous matrix, consisting of the polymer resp. polymers, but without any coating, obtainable according to the method of one of the preceding claims, wherein the matrix is sheet-like and suitable for wound dressing.

11. The biodegradable polymer/polymers for the use of claim 10, wherein the surface of the matrix is hydrophilised via a plasma treatment.

12. The biodegradable polymer/polymers for the use of claim 10 or 11, wherein the matrix has a porosity of at least 80 % or at least 90%.

13. The biodegradable polymer/polymers for the use of one of claims 10 to 12, wherein the nominal resorption time of the polymer resp. at least one of the polymers is less than 3 months.

14. The biodegradable polymer/polymers for the use of one of claims 10 to 13, wherein the polymer resp. polymers are selected from poly(α-hydroxy) carboxylic acids such as PLA and PLGA.

15. The biodegradable polymer/polymers for the use of one of claims 10 to 14, wherein the matrix until use is packaged in a bag having at least one microporous side, the pore size of which is 0.05 mm or less.

16. The biodegradable polymer/polymers for the use of one of claims 10 to 15, infiltrated with primary cells or cell lines, cell components or tissue components, for use in a method of skin reconstruction.

## Revendications

1. Procédé de fabrication d'une matrice poreuse pour le recouvrement de plaie en un polymère biodégradable ou un mélange de plusieurs polymères biodégradables, dans lequel des particules du ou des polymères sont mélangées avec des particules d'un solide hydrosoluble et une solution d'au moins une partie du ou des polymères dans un solvant non miscible avec l'eau, le mélange est placé sur un support perméable à l'eau, le solvant est évaporé et le solide est éliminé par lavage, après quoi la matrice lixiviée est séchée et emballée sans revêtement dans un sachet avec au moins un côté microporeux et y est stérilisée et hydrophylisée sous l'effet d'un plasma.

2. Procédé selon la revendication 1, dans lequel le support perméable à l'eau est disposé pour le lavage sur un socle dans un bain d'eau.

3. Procédé selon la revendication 1 ou 2, dans lequel le support perméable à l'eau est constitué d'une plaque de matière plastique hydrophile et résistant aux solvants, ayant des pores traversants.

4. Procédé de fabrication d'une matrice poreuse pour le recouvrement de plaie en un polymère biodégradable ou un mélange de plusieurs polymères biodégradables, dans lequel des particules du ou des polymères sont mélangées avec des particules d'un solide hydrosoluble et une solution d'au moins une partie du ou des polymères dans un solvant non miscible avec l'eau, le mélange est inséré dans un moule avec un bord amovible, le solvant est évaporé et le bord avec la masse adhérant à celui-ci est retiré du fond du moule et le solide est ensuite éliminé par lavage, après quoi la matrice lixiviée est séchée et emballée sans revêtement dans un sachet avec au moins un côté microporeux et y est stérilisée et hydrophylisée sous l'effet d'un plasma.

5. Procédé selon la revendication 4, dans lequel le bord amovible comprend un anneau métallique.

6. Procédé selon la revendication 4 ou 5, dans lequel le bord amovible présente une forme de gaine cylindrique ou de gaine tronconique.

7. Procédé selon l'une des revendications précédentes, dans lequel le lavage comprend l'apport continu d'eau et l'évacuation de saumure.

8. Procédé selon l'une des revendications précédentes, dans lequel le plasma est généré dans un mélange de gaz qui contient de l'H₂O₂ et dans lequel le mélange de gaz traverse le côté microporeux du sachet.

9. Procédé selon l'une des revendications précédentes, dans lequel les pores du sachet sont inférieurs à 0,05 mm ou inférieurs à 0,025 mm.

10. Polymère(s) biodégradable(s) pour l'utilisation dans un procédé de recouvrement de plaie et éventuellement de reconstruction cutanée sous la forme d'une matrice poreuse constituée du ou des plusieurs polymères biodégradables mais sans revêtement, pouvant être obtenue avec le procédé selon l'une des revendications précédentes, dans lequel/lesquels la matrice est plane et est appropriée pour le recouvrement de plaie.

11. Polymère(s) biodégradable(s) pour l'utilisation selon la revendication 10, dans lequel/lesquels la surface de la matrice est hydrophilisée par un traitement au plasma.

12. Polymère(s) biodégradable(s) pour l'utilisation selon la revendication 10 ou 11, dans lequel/lesquels la matrice présente une porosité d'au moins 80 % ou d'au moins 90 %.

13. Polymère(s) biodégradable(s) pour l'utilisation selon l'une des revendications 10 à 12, dans lequel/lesquels le temps de résorption nominal du ou d'au moins un des polymères est inférieur à 3 mois.

14. Polymère(s) biodégradable(s) pour l'utilisation selon l'une des revendications 10 à 13, dans lequel/llesquels le ou les polymères sont choisis parmi des acides poly(α-hydroxy)carboxyliques tels que PLA et PLGA.

15. Polymère(s) biodégradable(s) pour l'utilisation selon l'une des revendications 10 à 14, dans lequel/lesquels la matrice est emballée jusqu'à son utilisation dans un sachet avec au moins un côté microporeux dont la taille de pore est de 0,05 mm ou inférieure.

16. Polymère(s) biodégradable(s) pour l'utilisation selon l'une des revendications 10 à 15, infiltré(s) de cellules ou de lignées cellulaires primaires, de parties de composants cellulaires ou tissulaires, pour leur utilisation dans un procédé de reconstruction cutanée.
